# EUROPÄISCHE PATENTSCHRIFT

(11) **EP 3 132 228 B1**
(45) Veröffentlichungstag und Bekanntmachung des Hinweises auf die Patenterteilung: **30.01.2019**
(21) Anmeldenummer: 15722026.0
(22) Anmeldetag: 15.04.2015
(51) Int. Cl.: F41J 11/00, F41J 7/06, F41A 35/00, F41G 1/46

(54) **VORRICHTUNG FÜR EREIGNISDARSTELLUNGEN BEIM DUELL-SCHIESSEN**
DEVICE FOR EVENT REPRESENTATIONS IN DUEL SHOOTING
DISPOSITIF POUR LA REPRÉSENTATION DES ÉVÈNEMENTS LORS D'UN DUEL AU PISTOLET

(30) Priorität: 15.04.2014 DE 202014101791 U
(43) Veröffentlichungstag der Anmeldung: 22.02.2017
(73) Patentinhaber: Bayer, Reiner, 67705 Trippstadt (DE)
(72) Erfinder: Bayer, Reiner, 67705 Trippstadt (DE)
(74) Vertreter: Patentanwaltskanzlei Vièl & Wieske PartGmbB
(86) Internationale Anmeldenummer: PCT/DE2015/100158
(87) Internationale Veröffentlichungsnummer: WO 2015/158332

(56) Entgegenhaltungen:
- EP-A1- 0 669 512
- WO-A1-2009/135674
- DE-U1-202009 014 784
- DE-U1-202011 102 282
- US-A1- 2009 146 916
- US-A1- 2014 092 245

## Beschreibung

Die Erfindung betrifft eine Vorrichtung für Ereignisdarstellungen beim Duell-Schießen.
Die DE 74 40 222 U1 beschreibt einen Luftpistolenstand mit einem über Umlenkrollen geführten Zugseil, an dem ein zwischen der Abschußstelle und dem Kugelfang verfahrbarer Träger mit einer Halterung für eine Präzisions-Schießscheibe befestigt ist, wobei die Halterung auch für die Aufnahme einer Duell-Schießscheibe ausgeführt ist und über ein elektrisch angetriebenes Drehglied am Träger gelagert ist, da zwischen einer Schußabgabestellung mit sichtbarer Drehscheibe und einer Pausenstellung mit aus dem Blickfeld gedrehter Duellscheibe verschwenkbar ist.
Auch die DE 40 31 937 A1 beschreibt eine Schießanlage mit einer einzigen Schießscheibe, die insbesondere für das sportliche Duell-Schießen mit Luftdruckwaffen geeignet ist und welche auf dem Laufwagen einer Scheibenzuganlage um eine vertikale Achse drehbar gelagert ist. Über einen Mikroprozessor und eine kabellose Fernsteuerung ist der Elektromotor so steuerbar, daß die Zielscheibe in vorbestimmten Zeitabständen die für das Duell-Schießen erforderliche Drehung um jeweils 90° durchführt.
Aus der DE 20 2011 102 282 U1 ist eine Duell-Schießeinrichtung mit wenigstens zwei Schußvorrichtungen mit jeweils einer Quelle für elektromagnetische Strahlen und mindestens zwei Zieleinrichtungen jeweils mit wenigstens einem Fotodetektor für elektromagnetische Strahlen der Quellen der Schußvorrichtungen und einer über eine rücksetzbare Schalteinrichtung mit dem Fotodetektor verbundene Signalvorrichtung bekannt, wobei die Schalteinrichtungen so miteinander zusammengeschaltet sind, daß bei der Detektion elektromagnetischer Strahlen eines Fotodetektors eine Signalvorrichtung angesteuert wird und die Detektion als erster Treffer wahrnehmbar ist. Die Signalvorrichtung ist hier im Zentrum der Zielscheibe angeordnet.

Darüber hinaus ist aus der DE 20 2009 014 784 U1 ein Schießstandsystem zur automatisierten Durchführung von Schießwettkämpfen bekannt, das ein Anzeigesystem mit unterschiedlichen Anzeigebereichen zur Signalisierung des Zeitablaufs des Schießvorganges aufweist. Nachteilig bei diesen Vorrichtungen ist, daß jeweils eine Schießanlage umgerüstet werden muß, um das Duell-Schießen trainieren zu können.

Der Erfindung liegt die Aufgabe zugrunde, eine Vorrichtung für das Duell-Schießen zu schaffen, die kein aufwendiges Umrüsten einer Schießanlage erfordert.

Diese Aufgabe wird erfindungsgemäß dadurch gelöst, daß eine Visualisierungseinheit zur Ereignisdarstellung vorgesehen ist, die an einem beim Schießen sich in unmittelbarer Nähe des Schützen befindlichen Ausrüstungsgegenstand befestigbar ist und über die ein Ereignis im Wahrnehmungsbereich des Auges des Schützen darstellbar ist.

Es hat sich im Rahmen der Erfindung überraschend gezeigt, daß es ausreicht, eine Visualisierungseinheit im Wahrnehmungsbereich des Auges des Schützen anzuordnen, um dem Schützen die Schießperiode anzuzeigen. Wichtig ist hierbei, daß die Visualisierungseinheit nicht im Bereich zwischen dem Auge und der Zielscheibe angeordnet wird. Dies würde beim Zielen stören. Es ist völlig ausreichend, die Visualisierungseinheit so anzuordnen, daß sie gerade noch im Sichtbereich des Auges des Schützen ist oder als gebündeltes Licht (Laser) direkt auf einen Bereich der Zielscheibe projiziert wird. Die Schießperiode kann dem Schützen durch einen Farb- oder Helligkeitswechsel oder ein anderes Signal der Visualisierungseinheit angezeigt werden. Der Vorteil dieser Vorrichtung besteht darin, daß keine Umbauten an der Schießanlage erforderlich sind und auch ein außerhalb von Schießanlagen ein Training des Zielvorgangs erfolgen kann, beispielsweise in der Wohnung des Schützen. Gerade das für das Duell-Schießen so wichtige Training des periodisch wechselnden Zielens während einer Schießperiode und des anschließenden Wartens kann mit einer solchen Vorrichtung erfolgen.

Die Visualisierungseinheit kann an einem beim Schießen sich in unmittelbarer Nähe des Schützen befindlichen Ausrüstungsgegenstand, beispielsweise dem Zielscheiben-Beobachtungsglas oder dem Gehörschutz des Schützen befestigt werden.

Da beim Duell-Schießen ein Gehörschutz getragen wird und dieser es ermöglicht, die Visualisierungseinheit im Wahrnehmungsbereich des Schützen anzuordnen, ist diese Ausbildung der Erfindung kostengünstig realisierbar. Die Stromversorgung und die Schaltelektronik können im Bereich des Gehörschutzes angeordnet werden. Entsprechendes gilt für andere Ausrüstungsgegenstände, wie das Zielscheiben-Beobachtungsglas.

In diesem Zusammenhang ist es vorteilhaft, daß der Gehörschutz eine Buchse aufweist, in die ein Stecker der Visualisierungseinheit einsteckbar ist.

Der Gehörschutz kann dann einfach mit einer Visualisierungseinheit für das Duell-Schießen versehen werden. Wird diese nicht mehr benötigt, kann sie einfach von dem Gehörschutz entfernt werden.

Ebenfalls sinnvoll ist in diesem Zusammenhang, daß die Visualisierungseinheit an einem flexiblen Tragelement angeordnet ist.

Das flexible Tragelement kann beispielsweise ein flexibler Stab sein, wie er beispielsweise bei Mikrophonen verwendet wird. Dies ermöglicht es, die Visualisierungseinheit an die jeweiligen körperlichen Gegebenheiten des Schützen anzupassen. An dem der Visualisierungseinheit gegenüberliegenden Ende des flexiblen Tragelementes kann dann der Stecker zum Verbinden mit der Buchse des Gehörschutzes angeordnet sein.

Eine andere bevorzugte Ausbildung der Erfindung besteht darin, daß die Visualisierungseinheit an einer Schießbrille befestigbar ist.

Auch Schießbrillen werden beim Duell-Schießen häufig getragen. Es ist daher möglich, die Visualisierungseinheit an einer Schießbrille zu befestigen, beispielsweise an deren Blende, wobei auch hier die Visualisierungseinheit nicht im Bereich zwischen dem Auge und der Zielscheibe angeordnet wird, sondern gerade noch im Wahrnehmungsbereich des Auges.

Es ist zur Erfindung gehörig, daß die Visualisierungseinheit zusätzlich mit einem akustischen Signalgeber versehen ist.

Dieser akustische Signalgeber kann neben dem optischen Signal der Visualisierungseinheit dem Duell-Schützen die Schießperioden und die Wartezeiten signalisieren. Bei einer Anbringung der Visualisierungseinheit im Gehörschutz des Schützen kann dies in besonders einfacher Weise erfolgen.

Schließlich besteht eine Weiterbildung der Erfindung darin, daß in die Visualisierungseinheit ein Mikrophon und eine Auswerteelektronik integriert sind.

Mit einem derartigen Mikrophon kann der abgegebene Schuß erfaßt werden und in einer Auswerteelektronik die Zeitdauer zwischen dem Beginn der jeweiligen Schießperiode und der Schußabgabe ermittelt werden. Bei Verwendung von Zielscheiben mit elektronischer Treffererfassung kann die signalgebende Elektronik mit der Auswerteelektronik der entsprechenden Schießbahn gekoppelt werden.

Nachfolgend wird ein Ausführungsbeispiel der Erfindung anhand von Zeichnungen erläutert.

Es zeigen
- Fig. 1: eine erfindungsgemäße Visualisierungseinheit an einem Gehörschutz,
- Fig. 2: den Gehörschutz mit abgenommener Visualisierungseinheit.

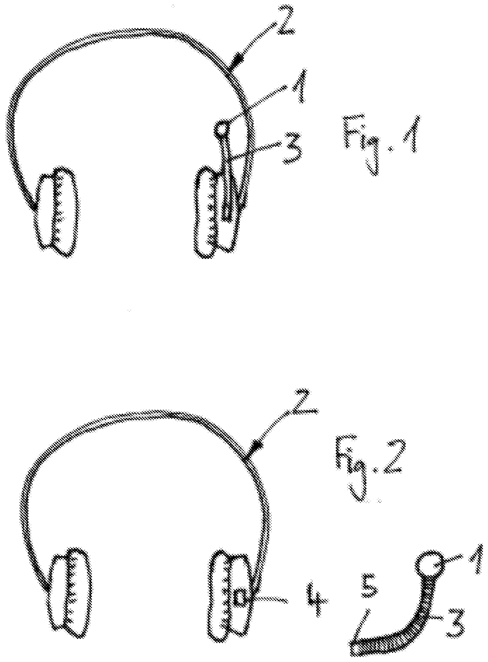

Wie in Fig. 1 dargestellt, ist eine Visualisierungseinheit 1 an einem Gehörschutz 2 für einen Schützen befestigt. Um die Visualisierungseinheit 1 an die Physiognomie des jeweiligen Schützen anzupassen, ist die Visualisierungseinheit an einem flexiblen Tragelement 3 angeordnet, so daß die Visualisierungseinheit 1 von dem Schützen nach seinen persönlichen Bedürfnissen positioniert werden kann.

Fig. 2 zeigt den Gehörschutz mit abgenommener Visualisierungseinheit 1, wobei letztere vergrößert dargestellt ist.

Im vorliegenden Fall weist der Gehörschutz 2 eine Buchse 4 auf, in die ein Stecker 5, der an dem der Visualisierungseinheit 1 abgewandten Ende des flexiblen Tragelementes 3 angeordnet ist, eingesteckt werden kann. Die Stromversorgung und die Schaltelektronik der Visualisierungseinheit 1 können im Bereich des Gehörschutzes 2 angeordnet werden, eine eventuell vorhandener zusätzlicher akustischer Signalgeber ebenfalls. Schließlich können auch ein Mikrophon und eine Auswerteelektronik in dem Gehörschutz angeordnet sein.

Will der Schütze das Duell-Schießen trainieren, steckt er den Stecker 5 der Visualisierungseinheit 1 in die Buchse 4 des Gehörschutzes 2, positioniert die Visualisierungseinheit 1 so, daß sie gerade noch im Gesichtsfeld liegt und schaltet - beispielsweise über eine Fernbedienung oder einen Schalter im Bereich des Gehörschutzes 2 - die Visualisierungseinheit 1 ein. Diese gibt dann anhand von Helligkeitswechseln, Farbsignalen oder anderen Signalen - eventuell unterstützt durch ein gleichzeitiges akustisches Signal - die Schießperioden und die Wartezeiten vor. Über das Mikrophon kann der abgegebene Schuß detektiert und mittels der Auswerteelektronik die jeweils vergangene Zeit zwischen dem Beginn der Schießperiode und der Schußabgabe ermittelt werden.

Die Duell-Trainingseinheit wird also in unmittelbarer Nähe des Schützen montiert und betrieben. Somit kann das Duelltraining jederzeit und an jeder beliebigen Schießbahn durchgeführt werden, ohne daß ein anderer, danebenstehender Schütze gestört wird.

## Patentansprüche

1. Vorrichtung für Ereignisdarstellungen beim Duell-Schießen, wobei eine Visualisierungseinheit (1) zur Ereignisdarstellung vorgesehen ist, über die ein Ereignis im Wahrnehmungsbereich des Auges des Schützen darstellbar ist, **dadurch gekennzeichnet, dass** die Visualisierungseinheit (1) an einem beim Schießen sich in unmittelbarer Nähe des Schützen befindlichen Ausrüstungsgegenstand befestigbar ist.

2. Vorrichtung gemäß Anspruch 1, **dadurch gekennzeichnet, daß** der Ausrüstungsgegenstand ein Zielscheiben-Beobachtungsglas ist.

3. Vorrichtung gemäß Anspruch 1, **dadurch gekennzeichnet, daß** der Ausrüstungsgegenstand ein Gehörschutz des Schützen ist.

4. Vorrichtung gemäß Anspruch 3, **dadurch gekennzeichnet, daß** der Gehörschutz (2) des Schützen eine Buchse (4) aufweist, in die ein Stecker (5) der Visualisierungseinheit (1) einsteckbar ist.

5. Vorrichtung gemäß Anspruch 4, **dadurch gekennzeichnet, daß** die Visualisierungseinheit (1) an einem flexiblen Tragelement (3) angeordnet ist.

6. Vorrichtung gemäß Anspruch 1, **dadurch gekennzeichnet, daß** die Visualisierungseinheit (1) an einer Schießbrille befestigbar ist.

7. Vorrichtung gemäß einem der Ansprüche 1 bis 6, **dadurch gekennzeichnet, daß** die Visualisierungseinheit (1) zusätzlich mit einem akustischen Signalgeber versehen ist.

8. Vorrichtung gemäß einem der Ansprüche 1 bis 7, **dadurch gekennzeichnet, daß** in die Visualisierungseinheit (1) ein Mikrophon und eine Auswerteelektronik integriert sind.

## Claims

1. Device for representing events in duel shooting, wherein a visualizing unit (1) is provided for representing events via which an event can be represented in the perception region of the markman's eye, **characterised in that** the visualizing unit (1) is fastenable to a piece of equipment in the immediate vicinity of the marksman during shooting.

2. Device according to claim 1, **characterised in that** the piece of equipment is a target viewing glass.

3. Device according to claim 1, **characterised in that** the piece of equipment is a hearing protector of the marksman.

4. Device according to claim 3, **characterised in that** the marksman's hearing protector (2) has a socket (4) into which a plug (5) of the visualizing unit (1) can be inserted.

5. Device according to claim 4, **characterised in that** the visualizing unit (1) is mounted on a flexible support element (3).

6. Device according to claim 1, **characterised in that** the visualizing unit (1) can be fastened to shooting glasses.

7. Device according to any one of the claims 1 to 6, **characterised in that** the visualizing unit (1) is provided additionally with an acoustic signal transmitter.

8. Device according to any one of the claims 1 to 7, **characterised in that** a microphone and evaluating electronics are integrated in the visualizing unit (1).

## Revendications

1. Dispositif pour la représentation d'événements lors du tir duel au pistolet, une unité de visualisation (1) pour la représentation d'événements étant prévue, par l'intermédiaire de laquelle un événement peut être représenté dans la zone de perception de l'oeil du tireur, **caractérisé en ce que** l'unité de visualisation (1) peut être fixée à un équipement situé dans le voisinage immédiat du tireur pendant le tir.

2. Dispositif selon la revendication 1, **caractérisé en ce que** l'équipement est un verre d'observation de cible.

3. Dispositif selon la revendication 1, **caractérisé en ce que** l'équipement est un protecteur auditif du tireur.

4. Dispositif selon la revendication 3, **caractérisé en ce que** le protecteur auditif (2) du tireur a une prise (4) dans laquelle une fiche (5) de l'unité de visualisation (1) peut être enfichée.

5. Dispositif selon la revendication 4, **caractérisé en ce que** l'unité de visualisation (1) est disposée sur un élément porteur flexible (3).

6. Dispositif selon la revendication 1, **caractérisé en ce que** l'unité de visualisation (1) peut être fixée sur des lunettes de tir.

7. Dispositif selon l'une des revendications 1 à 6, **caractérisé en ce que** l'unité de visualisation (1) est en outre munie d'un émetteur de signal acoustique.

8. Dispositif selon l'une des revendications 1 à 7, **caractérisé en ce qu'**un microphone et une électronique d'évaluation sont intégrés dans l'unité de visualisation (1).
